# EUROPEAN PATENT APPLICATION

(11) **EP 4 641 321 A1**
(43) Date of publication of application: **29.10.2025**
(21) Application number: 24171769.3
(22) Date of filing: 23.04.2024
(51) Int. Cl.: G05B 17/02

(54) **CUSTOMIZED RAMAN MODELLING**

(71) Applicant: Sartorius Stedim Data Analytics AB, 903 33 Umeå (SE)
(72) Inventor: HÖHSE, Marek, Göttingen (DE); SUROWIEC, Izabella, Umeå (SE); GRIMM, Christian, Göttingen (DE); Schmidberger, Timo, Göttingen (DE)
(74) Representative: Müller-Boré & Partner Patentanwälte PartG mbB

(57) **Abstract**

A computer-implemented method (100) for generating a spectral model for controlling and/or monitoring a process to produce a chemical, biopharmaceutical or biotechnological product, comprising: - providing (S120) a database (202), the database (202) storing plural observation data sets associated with respective observations of chemical, biopharmaceutical or biotechnological past processes, each of the observation data sets comprising: stored spectral trajectory data, one or more stored process descriptors, and at least one corresponding actual analytical measurement of a process parameter recorded during performance of a respective past process; - obtaining (S140), from a user, user-based input data comprising at least one of the following: input spectral trajectory data and one or more input process descriptors; - determining (S160) a subset of the stored spectral trajectory data in accordance with at least one selection criterium by querying the database (202) using the user-based input data; and - using (S180) the subset of the stored spectral trajectory data to generate the spectral model providing process parameter values for a whole duration of an execution of the process.

## Description

The technical field of the present disclosure relates to a computer-implemented method for generating a spectral model for controlling and/or monitoring a process to produce a chemical, biopharmaceutical, or biotechnological product. The present disclosure further relates to a computer program product comprising computer-readable instructions, which, when loaded and executed on a computer system, cause the computer system to perform operations according to the method, and to a computer system operable to control and/or monitor a process to produce a chemical, biopharmaceutical, or biotechnological product.

Examples of processes according to the present disclosure are industrial processes, particularly biopharmaceutical processes, for example a process for monoclonal antibody (mAb) production. A process of the present disclosure may involve chemical conversion of material in conjunction with the transfer of mass, heat, and/or energy. The process particularly may be scale dependent; in other words, the process may behave differently on a small scale (e.g., in a laboratory) in comparison to a large scale (e.g., in production). The process may include heterogeneous chemical reactions.

The process of the present disclosure may be a batch process, which is an example of an industrial, biopharmaceutical process. The batch process may involve producing small amounts of a product, and gradually or stepwise scaling up to larger amounts. The batch process may involve chemical and/or biological reactions that take time to complete. In batch processing, multiple batches may be produced, possibly at different scales (e.g., about five liters, about ten liters, about one hundred liters). There may be a pause between each batch, e.g., to set up a new batch.

The process of the present disclosure may be a fed-batch process. In contrast to the simple batch process, the growth rate can be regulated in a fed-batch process. The process may be carried out in a bioreactor designed to accommodate increasing volumes. The production system of the fed batch process particularly may always be at a quasi-steady state. One or more nutrients may be fed into the bioreactor during cultivation and the product may remain in the medium until the end of the run. The fed batch process may involve a culture in which a base medium supports initial cell culture and a feed medium is added to prevent nutrient depletion. The base medium and the feed medium may be considered parts of the medium in the vessel.

The process of the present disclosure may also be a perfusion or a continuous process.

In a continuous process, a portion of the mixed culture broth, i.e., medium and cells, is harvested, which substantially results in a constant cell density. In a perfusion process, only the medium is harvested and the cells are kept in the culture by a cell retention device. The process may be designed such that growth is limited by the availability of one or two (or more) limiting components of a medium. The growth is dependent not only on the external components in the medium, but also on what cells excrete to it (what in fed-batch process is considered the main cause of cell death). For example, in a perfusion process needed components may be added to medium, but at the same the old medium may be at least partially exchanged (in a controlled way) together with product harvest via harvest line.

Development of a new production process is time consuming and expensive, mainly due to the number of operating conditions and changes in the process that influence its final outcome. This limitation is especially difficult to overcome for smaller companies that have determined a product to be produced and some or all of the quality attributes of the product, but do not know an approach that can be used to successfully monitor and control critical process parameters and product attributes.

Spectroscopy techniques have been implemented in process monitoring within the biopharmaceutical industry for a long time due to their non-destructive ability to measure multiple analytes simultaneously and possibility for inline or online connection to the process. Especially monitoring of mammalian cell cultures with Raman, NIR and UV spectroscopy and chemometric tools has been well demonstrated and documented. The main metabolites and nutrients of a cell culture, as well as several cell characteristics and critical quality attributes (CQAs), can be predicted using spectral data and partial least square (PLS) models with root mean square error of prediction (RMSEP) quite close to off-line reference measurement accuracy. Real-time monitoring of critical process parameters (CPPs) has been also used to implement a glucose feeding control loop, leading to improved productivity. These results demonstrate that spectroscopy methods can be efficiently used to monitor cell cultures in real-time and in situ, automate processes and even open the door to the real-time release (RTR) of the batches.

The present disclosure addresses challenges faced when developing a model (for CPPs, CQAs, and/or other attributes) for process monitoring and/or control using spectral data. Development of such a model is a huge effort that requires a substantial amount of time, as a user needs to start acquiring data in small scale bioreactors, e.g. from 15 ml to 20 L, especially 15 ml, 250 ml, 1L, 2L, 5L, 10L, 20L, and then gradually move towards higher scale bioreactors, e.g. 50 L, 100L, up to 1.000 L or 2.000 L. Development of spectral models with an acceptable performance in terms of accuracy and precision is also very complex, as users need to test multiple conditions and design options. Application of spectral and other relevant data generated from previous processes could support model development, but a technical solution on how to do it is still missing. Another problem is that implementation of any solution would require access to an extensive database of processes (with spectral data recorded for them), which typically is not achievable for the majority of companies.

The majority of work done so far focuses on model building based on the same Raman analyzer hardware and in the same, narrow laboratory settings. As such, the key issues when using Raman spectroscopy in biopharmaceutical environments are not addressed by the models that are built so far: instrumental variability, variability between different labs, process scales, types (fed-batch, continuous feed, perfusion, etc.), cell lines and media, products, etc. That is why such models often have limited applicability and cannot be easily used for new processes, scales, etc.

According to one aspect, it is an object of the invention to allow for generation of a spectral model for controlling and/or monitoring a process to produce a chemical, biopharmaceutical, or biotechnological product, wherein it is possible to generate a reliable spectral model with enhanced applicability specific to a chemical, biopharmaceutical, or biotechnological process.

This object is achieved by the subject matter of the independent claims. Particular embodiments can be derived from the dependent claims.

According to an aspect of the invention, a computer-implemented method for generating a spectral model for controlling and/or monitoring a process to produce a chemical, biopharmaceutical, or biotechnological product, is provided, the method comprising:
providing a database, the database storing plural observation data sets associated with respective observations of chemical, biopharmaceutical, or biotechnological past processes, each of the observation data sets comprising stored spectral trajectory data, one or more stored process descriptors , and at least one corresponding actual analytical measurement of a process parameter recorded during performance of a respective past process;
obtaining, from a user, user-based input data comprising at least one of the following: input spectral trajectory data, and one or more input process descriptors;
determining a subset of the stored spectral trajectory data in accordance with at least one selection criterium by querying the database using the user-based input data; and
using the subset of the stored spectral trajectory data to generate the spectral model providing process parameter values for a whole duration of an execution of the process.

A database is provided that stores plural observation data sets associated with respective observations of chemical, biopharmaceutical, or biotechnological past processes, each of the observation data sets comprising stored spectral trajectory data, one or more stored process descriptors, and at least one corresponding actual analytical measurement of a process parameter recorded during performance or execution of a respective past process. Accordingly, the database particularly stores different data from past processes that were already run. These past processes are distinct from the process that is to be controlled and/or monitored by using the spectral model that is to be generated by the method. In other words, a past process is not the same process as the process that is to be controlled and/or monitored. The process that is to be controlled and/or monitored is a future process that is different from the past process on a temporal scale. In particular, the observations data sets may be stored in the database at a timepoint that precedes the timepoint at which the process to be controlled and/or monitored starts running. Thus, the database particularly stores historical data which are used to generate a spectral model for controlling and/or monitoring a future process.

The database may be a relational database in which the data are stored in related tables. The communication can take place for example by means of a database query language (e.g., Structured Query Language SQL). The database may be hosted on a server system and/or may be accessible to users via web access, e.g., a cloud. The database may reside on a server cluster which is under the control of a provider.

The database stores observation data sets comprising stored spectral trajectory data. Spectral trajectory data particularly are or comprise spectral data or spectra, e.g., Raman, IR, fluorescence, and/or UV-Vis spectra, with associated time information. For example, the associated time information may be information about the process maturity, i.e., process maturity information. Such spectral trajectory data are beneficial for creating a model, i.e., a spectral model, wherein the spectra may be used as input data (x value) and maturity and/or time is the output or target value (y-value).

Thus, a trajectory may be a time-based profile of measurements recorded during performance or execution of the respective process. In other words, the trajectory may be understood to summarize and provide an overview of the associated process. The trajectory may be implemented as a curve or graph that describes an associated process over time. The trajectory may also be referred to as a control chart (or a batch control chart in the context of an associated batch process). In the context of a batch process, each batch may have its own corresponding trajectory.

The database specifically is an extensive database that may store spectral trajectory data from different types of processes, as well as one or more process descriptors such as one or more of the following: process type, media platform, product, Chinese Hamster Ovary (CHO) cell line, scale, etc. More generally, the process descriptors may comprise information describing one or more characteristics of the process, in particular characteristics that may be known a *priori,* i.e., before running the process, such as characteristics concerning the setup of the process. Thus, the process descriptors may comprise information about features of the process that is not measured during the process or otherwise derived from the running or completed process. For instance, the process descriptors may be all the metadata that help to describe the way the process is run and anything else relevant for this process.

The process descriptors may be relevant metadata describing the context of spectral data origin, e.g., cell line, media, media composition, and/or strain, etc. The relevant metadata may further provide information about scale, medium platform, and/or feeding strategy, etc. In particular, the process descriptors might provide information on one or more of the following: equipment used for running the bioprocess, scale of the process, type of the process, type and/or name of the product and/or biological systems used to produce it, media system used, etc. The process descriptors may be set or input by the user.

The database may be accessed by multiple users. The database may store spectral data from different bioprocesses. Besides the time evolution data from a substantial part of the process or the whole process, i.e., the spectral trajectory data, the database may store spectral data without time evolution, i.e. single point spectral data. In the context of a process, each process batch (or perfusion experiment) may have its own corresponding trajectory. Thus, the database content may cover CHO-based protein production from different media platforms, cell lines, product types, types of processes (batch, perfusion), and/or process scales, etc.

The observation data sets may further comprise at least one corresponding actual analytical measurement of a process parameter recorded during performance of a respective past process. The actual analytical measurement or measurements of a process parameter particularly refer to data that is measured during a respective past process. Accordingly, "actual analytical measurements of a process parameter" are actually measured, while the process parameter values provided by the spectral model described herein are not measured but rather are derived from spectral data.

Analytical measurements of a process parameter may be data acquired, e.g., with, usually off-line, analyzers, that are not spectral, e.g., levels of glucose or other compounds in the cell medium (like for example ammonia, salts, and/or dissolved oxygen), cell parameters (like for example viable cell density, total cell density, cell viability, and/or cell diameter etc.), process performance parameters (like for example titer) and product quality attributes (like for example glycosylation, charge variants, and/or aggregates, etc.).

Thus, the database may contain spectral data with relevant analytical measurements of a process parameter, e.g., analytical measurements of process parameters and/or product quality attributes, and one or more process descriptors, wherein the one or more process descriptors may be used to pre-select relevant spectra for the model, as explained below.

Analytical measurements may be or may comprise additional process measurements (CPP, CQA, process KPI) from measurement instruments, e.g., one or more sensors, one or more analyzers, etc., achieved during the past process by in-line, on-line, or at-line measurements, e.g., pH, titer, VCD, intact mass, temperature, dissolved oxygen (DO), media composition, osmolality, etc.

At-line measurements may be measurements where the sample is removed, isolated from, and analyzed near to the process stream. In-line measurements may be measurements where the sample is not removed from the process stream and can be invasive or non-invasive. On-line measurements may be measurements where the sample is diverted from the manufacturing process and may be returned to the process stream.

For a continuous upstream process such as perfusion, continuous in-line sampling and/or testing can be performed to ensure that process inputs particularly are maintained within target ranges. The analytical needs for continuous upstream processing are relatively well defined using in-line spectroscopic approaches to determine bioreactor conditions and/or product quantity, while rapid in-line product quality measurements remain a significant gap. On-line product quality testing at defined intervals could supplement in-line analytical outputs ensuring product quality (such as the charge or glycosylation profile) is maintained.

In a well-defined process, adjustments to processing conditions may be performed to compensate for the on-/at-line analytical output(s) as needed. For continuous downstream processing (DSP), the challenge is considerably higher, since no in-line instrumentation currently exists to monitor purity and/or charge or size heterogeneity to demonstrate a successful unit operation.

Accordingly, the database particularly stores historical data of spectral trajectories, one or more process descriptors, and/or corresponding actual analytical measurements of a process parameter. Optionally, the database may also store process parameters. This allows for development of spectral models for process controlling and/or monitoring based on access to extensive historical data. This may be beneficial for a majority of users who otherwise would not have access to extensive historical data. In particular, a wide number of different processes may be used as basis of the database, which will result in more robust models that will be easily transferred between scales and/or different types of processes, for example, a model developed in intensified fed-batch may be applied in a perfusion process.

User-based input data (directly or indirectly) are obtained from a user, wherein the user-based input data comprise at least one of input spectral trajectory data and one or more input process descriptors. In particular, the user-based input data might comprise input spectral trajectory data and one or more input process descriptors. However, it would be also possible that the user-based input data comprise input spectral trajectory data and no input process descriptors. Obtaining both input spectral trajectory data and one or more input descriptors would be advantageous, since the subset of stored spectral trajectory data may be determined that better reflects the conditions of the process that is considered to be controlled and/or monitored. Also, a more robust spectral model will be ensured in case of both input spectral trajectory data and one or more input descriptors.

Similar to the stored spectral trajectory data, the input spectral trajectory data particularly are or comprise spectral data or spectra, e.g., Raman, IR, fluorescence, or UV-Vis spectra, with associated time information. For example, the associated time information may be information about the process maturity, i.e., process maturity information. Such spectral trajectory data are beneficial for creating a model, i.e., a spectral model, wherein the spectra are used as input data (x value) and maturity and/or time is the output or target value (y-value). Thus, a trajectory may be a time-based profile of measurements recorded during performance or execution of the respective process. In other words, the trajectory may be understood to summarize and provide an overview of the associated process. The trajectory may be implemented as a curve or graph that describes an associated process over time. The trajectory may also be referred to as a control chart (or a batch control chart in the context of an associated batch process). In the context of a batch process, each batch may have its own corresponding trajectory.

The user-based input database may comprise spectral trajectory data from different types of processes, as well as one or more input process descriptors such as process type, media platform, product, CHO cell line, and/or scale, etc. As already explained above, input process descriptors may be all the metadata that help to describe the way the process is run and anything else relevant for this process, like for example description of equipment used for running the bioprocess, scale of the process, type of the process, type and name of the product and biological systems used to produce it, media system used, etc.

The user-based input data may be obtained from multiple users. The user-based input data may comprise spectral trajectory data from different bioprocesses. Besides the time evolution data from a substantial part of the process or the whole process, i.e., spectral trajectory data, the user-based input data may further comprise single point spectral data. In the context of a process, each process batch (or perfusion experiment) may have its own corresponding trajectory. Thus, the content of the user-based input data may cover CHO-based protein production from different media platforms, cell lines, product types, types of processes (batch, perfusion), and/or process scales, etc.

As outlined before, the user-based input data (directly or indirectly) are obtained from at least one user. In particular, the user may set the one or more input process descriptors. The input process descriptors may comprise similar or the same process descriptors as outlined above with respect to the stored process descriptors. Setting one or more input process descriptors may help the user to define the process in which the user is interested.

Also, the user may provide input spectral trajectory data. In order to obtain these input spectral trajectory data, at least one run of the desired process may be performed. However, it may not be necessary to run said desired process until its end. The process may be interrupted after some time, in particular after having sufficient information on the process that is desired. Interrupted may have the meaning of stopping the process and not going on with running the same process after some time. In this respect, the process that is run and interrupted is different from the future process that is to be controlled and/or monitored by the generated spectral model. Rather, the interrupted process is only used for providing and/or obtaining the input spectral trajectory data that are used in the subsequent step of determining a subset of the stored spectral trajectory data. By doing so, the spectral model that is to be generated in a subsequent step may be used for a whole duration of an execution of one or more future processes.

Obtaining, from at least one user, user-based input data allows for selection of very relevant (possibly most relevant) stored spectral trajectory data for generating a spectral model as outlined below. Furthermore, a proper spectra pre-processing, as well as generation of validated model that is suitable for process control and/or monitoring is ensured. Such an improved selection of the relevant stored spectral trajectory data for the generation of the spectral model may be obtained by, for example, phase comparison, spectra pre-processing, and/or application of several search algorithms together.

After providing the database that stores plural observational data sets and after obtaining the user-based input data, a subset of the stored spectral trajectory data is determined in accordance with at least one selection criterium by querying the database using the user-based input data.

The method particularly is based on a mathematical algorithm for selection of most similar stored spectral trajectory data from the database, wherein the algorithm may select most relevant spectral trajectory data for the spectral model that will be generated.

Accordingly, the step of determining a subset of the stored spectral data in accordance with at least one selection criterium by querying the database using the user-input data could be done by using the input spectral trajectory data. For example, this would lead to a so-called Batch Evolution Model (BEM) which could be compared to existing ones in a similarity.

Stored spectral trajectory data may be selected with the aim to generate a spectral model, e.g., a multivariate spectral model, which can be used for controlling and/or monitoring the process. The applied selection algorithm(s) will depend on the type and/or number of user-based input data. To enable selection of most suitable stored spectral trajectory data for the spectral model, user may provide information as user-based input data for the selection algorithm and query the database using these user-based input data.

According to an example, querying the database using the user-based input data may be based on comparing spectral trajectory data from a finished biopharmaceutical process stored in the database, i.e., from a past or historical process, with the user-based input spectral trajectory data that may be derived from at least parts of a process that the user already has run. Accordingly, time dependent data are compared with each other.

When querying the database using time-dependent data, the user may provide user-based input spectroscopy batch data of several cultivations, e.g., N cultivations, and/or additional relevant process descriptors. The input spectroscopy batch data may be data with a time component, i.e., input spectral trajectory data. Accordingly, relevant spectral trajectory data from the database that are used for generating the spectral model may be obtained by comparison of input spectral process trajectories obtained from the user with the spectral process trajectories stored in the database.

Spectral pre-processing can be used to improve database search, e.g., Asymmetric Least Square Correction (AsLS), Standard Normal Variate (SNV), Multiplicative Signal Correction (MSC), Derivatives, peak area, peak height and/or water band normalization and/or Multivariate Curve Resolution (MCR) to deconvolute signal of known media components like glucose, glutamine from the rest of the spectrum and then comparing deconvoluted spectral 'backgrounds'. Several pre-processing and/or selection algorithms can be used in parallel to improve robustness of the selection.

In order to obtain input spectral trajectory data from the user, which is subsequently used to query the database, the user may already have performed a bioreactor run, e.g., in a multi-parallel bioreactor system, and may have recorded Raman spectra over at least some time through this bioreactor run with the corresponding glucose values. In other words, the user does not need to fully run the desired bioreactor process. Rather, the user may interrupt or stop this bioreactor process after some time when having sufficient information for creating input spectral trajectory data. Instead of fully running the bioreactor process, the user may use the database to complement the available data with one or more data from the database to have a ready-to-use spectral model, e.g., a Raman calibration model, e.g., for glucose concentration prediction in the user's bioprocess.

The user may input the Raman spectral trajectory data and/or one or more input process descriptors to the database. The one or more process descriptors may be process scale, equipment used to run the bioprocess, type of Raman analyzer, type of process (e.g., fed-batch, perfusion), name of the product, media type, and/or cell line type.

The database algorithm may use descriptor(s) given by the user to pre-select a set of spectral trajectories from the database. The algorithm particularly may select only the ones coming from the same media and/or product type used by the user.

Thus, one or more database descriptors from the user may be used for a pre-selection of spectral trajectory data in the database. For example, the additional metadata may be used for a narrowed selection to relevant media platform, cell line, type of process, and/or scale etc. In particular, the relevant spectral trajectory data for the spectral model may be selected by a comparison of process trajectories for specific phases of the process, e.g., cell development stages, rather than for the whole process. This particularly is both to improve model performance by building it on most relevant spectral trajectory data, but also to overcome difficulties connected to comparing process trajectories of different dynamics, e.g., different total process times, different switch points between process phases, different feeding strategies, etc.

Then, spectral trajectory data may be processed, with one or several methods and process time-trajectory comparison algorithms applied to fine-tune the spectral selection to a number of spectral trajectories closest to the user's spectral trajectory data. These spectral trajectory data, e.g. with connected glucose values, together with the spectral trajectory data from the user may then be included in the spectral model, e.g., the Raman calibration model, e.g., for prediction of glucose concentration. This model may then be applied or output, e.g., provided to the user.

According to a different concept, there is disclosed a method wherein the data that are compared with each other is not time dependent. In particular, the user-based input data that are to be compared with the data stored in the database may be based on a medium before the biopharmaceutical process starts or a search spectrum at a given point of time of a biopharmaceutical process that is used as input for the database. According to the different concept, the user may provide spectral data of a medium at a given point of time before or after inoculation, with relevant information about the users desired process or any other relevant metadata. Metadata may, e.g., include cell strain, product name, and/or batch type. Thus, the data may be data without a time component. It may be possible, that the user may provide spectral data of a medium before or after feed.

According to the different concept, the user does not need to have spectral batch data. Rather, data without time component are possible. Also, a relevant spectral set for the generation of the spectral model may be selected from the database using similarity search between input spectrum or input spectra and spectra in the database. Possible methods for similarity search that can be applied may be spectral similarity measure algorithms, Principal Component Analysis (PCA), Dendrogram/ Mahalanobis distance, Euclidian distance, SVMs, random forests, etc. Additional metadata given by the user can be used for pre-selection of spectral data, i.e., narrowing selection to relevant media platform, cell line, type of process, scale etc. Spectral pre-processing can be used to improve database search, e.g., AsLS, SNV, MSC, derivatives, peak area, peak height and/or water band normalization or Multivariate Curve Resolution (MCR) to deconvolute signal of known media components like glucose, glutamine from the rest of the spectrum and then comparing deconvoluted spectral 'backgrounds'.

Several pre-processing and selection algorithms can be used in parallel to improve robustness of the selection. According to the different concept, the user may use one or few Raman spectra at a particular point in time from his medium and/or process to obtain a spectral model, e.g., a Raman based model for glucose prediction in the user's process. The user may submit the spectrum to the database, together with one or more descriptors of his process, for example, a bioprocess scale, equipment used for the process, type of Raman analyzer, type of process (e.g., fed-batch), name/class of the product (e.g., lgG1), media type (e.g., Cellca platform), and/or cell line type (e.g., CHO DG44).

According to the aspect of the invention, a database algorithm may do the pre-sorting of the spectra that are in the database based on given process descriptors. For example, the database algorithm may preselect spectra only gathered for a CHO process producing IgG1 product on Cellca platform. Then, the algorithm may compare the input spectrum with the spectra in the database selecting most similar spectra particularly based on a principal component analysis (PCA) similarity search and may provide the user with a set of spectra with related glucose values and/or a multivariate model built on them for prediction of glucose. Several spectra pre-processing algorithms may be applied or tested before similarity search is done. Also, several similarity searches may be tested as well.

The querying according to the aspect of the invention allows for selecting most similar spectral trajectory data in accordance with at least one selection criterium. Accordingly, a subset of the stored trajectory data may be determined based on using the user-based input data, i.e., at least one of input spectral trajectory data and one or more input process descriptors, as described further below. Thus, a fine-tuned selection of stored spectral trajectory data for generating the spectral model is ensured.

In a next step, a spectral model may be generated by using the selected subset of stored spectral trajectory data, wherein the spectral model provides process parameter values for a whole duration of an execution of the future process. This spectral model may be provided to the user for monitoring and/or control of the desired process. In particular, the desired process parameter and, preferably quality attribute(s), may be monitored and/or controlled by the generated spectral model.

Once similar data have been selected from the database based on the user-based input data, these data can be processed to generate a spectral calibration model. Accordingly, a spectral calibration model such as a Raman-based monitoring model may be obtained. Such a calibration model may be a partial least squares (PLS) model, although other approaches can be used as well, e.g., a multiple linear regression model. In particular, partial least squares (PLS) and/or multiple linear regression modeling methods may be used to correlate the Raman spectra to the analytical measurements of a process parameter.

To build the calibration model, spectra may be processed with one or a combination of several spectral pre-processing methods, e.g., AsLS, SNV, MSC, derivatives, peak area, peak height, and/or water band normalization. Multivariate Curve Resolution (MCR) can be used to deconvolute signal of known media components like glucose, glutamine from the rest of the spectrum and this way facilitate comparison of deconvoluted spectral 'backgrounds' for improved similarity search. So, the spectra pre-processing can be used both to improve database search, but is also advantageous to obtain good spectral models. To improve the models, only part(s) of the spectrum may be selected, for example only bands corresponding to the Raman signal of the molecule which concentration is to be predicted may be included. The most relevant part(s) of the spectrum for model building can also be obtained with the application of different variable selection methods. Processing of spectra could involve taking a part of a spectrum, and this part can be decided based on process knowledge or applying some variable selection approach.

In addition, in model building, different scaling can be applied to the data, for example centering may be used for spectral models.

According to the present method, a model based on spectral data, e.g., the input spectral trajectory data obtained from the user and selected stored spectral trajectory data from the database, is provided that may be used to provide behavior of process parameter values for a whole duration of an execution of a future process. Also, process parameters and/or product attributes may be predicted or obtained by the model. A spectral model is generated, which may be provided to a user.

In particular, a global generic spectral model advantageously is generated, i.e., a spectral calibration model that is not a local spectral calibration model which is used to obtain a response related to the spectrum that is used as input for database search. In particular, using an entire data set to predict a new measurement point is called a global model and, just like any model, it needs to balance robustness and accuracy. An "entire data set" may mean that the entire time course of the data set is used, and not just a single time point or single time interval of the data set, to predict a new measurement point. According to the present disclosure, an entire observation data set of the database and the entire user-based input data of past processes particularly are used to generate a spectral model predicting in the sense of providing process parameters values for a whole duration of an execution of a new (future) process. An "entire observation data set" of the database and "entire user-based data" of past processes may mean that the entire time course of the observation data set or user-based data is used, respectively, and not just a single time point or single time interval of these data to generate the spectral model.

A different approach, which is in contrast to the present disclosure and is only described for better understanding the invention, is to use only the local data, i.e., not the entire data set, based on a similarity or distance approach, to predict a new measurement point. Using only the local data may mean that a single time point or single time interval of these data may be used to predict a new measurement point. This approach is called a local model. Local models provide an alternative to global models because they can dynamically respond to process conditions, account for process drift, and simplify model maintenance. Such a local model may only provide process parameter values for the same process and may not provide process parameter values for a whole duration of an execution of a new (future) process. For example, just-in-time learning (JITL) may be integrated in order to develop local models of glucose, lactate, glutamine, glutamate, calcium, sodium, viability, and viable cell density (VCD).

In other words, the global generic spectral calibration model is rather used for future prediction in a separate experiment or process, and the input spectrum(a) is not queried to predict output response related to this input spectrum(a). The global generic spectral calibration model may be used for monitoring and/or controlling specific process parameter(s), e.g., glucose concentrations or titer. This is in contrast to a local spectral model, which is used for prediction in the same experiment and process and is not used for future prediction in a separate experiment.

In view of the aspect of the invention it is noted that all statistical descriptors, performance data etc. and also the data of the plural observation data sets of the database and the user-based input data are determined and accessible for the user before running the process. In other words, the data are determined a *priori.* This generic approach is different from a local approach which might provide the data on the fly during running the process (e.g., by taking a current value of the spectra or other source and from one or more time intervals before current time to predict the next values of the process) and the data are not determined or determinable before the process is running. In contrast to the local approach, the generic approach takes the whole process evolution into account, e.g., by using a database with stored time dependent trajectory data and, optionally, input spectral trajectory data.

The method for generating a spectral model for controlling and/or monitoring a process, in particular a process by a spectroscopy system such as a Raman spectroscopy system, to produce a chemical, biopharmaceutical, or biotechnological product, allows for an application of wide number of different processes as basis of the database, which will result in more robust models that will be easily transferred between scales and/or different types of processes, for example a model developed in an intensified fed-batch process may then be applied in a perfusion process. A new production process may be developed and the method can be used to successfully monitor it and/or control critical process parameters and/or product attributes in a time- and cost-efficient manner. The method further allows for an improved selection of the relevant spectral data for the generation of the spectral model, e.g., obtained by phase comparison, spectra pre-processing, and/or application of several search algorithms together. This results in an improved output spectral model that provides process parameter values for a whole duration of an execution of the process.

In summary, the method according to one aspect of the invention allows for an improved selection of the relevant spectral data for generation of more robust spectral models for process monitoring and/or control, particularly multivariate spectral models, generated without any (or with only a limited) set of experimental data from the user side. Alternatively, or additionally, the method according to one aspect of the invention allows for an easier transfer of spectral models between process steps, scales, and/or types of processes. The model allows for faster and less expensive process development resulting in faster release of a chemical, biopharmaceutical or biotechnological product, and an improved monitoring and/or control of the bioprocess by the user. Thus, a technical solution is provided that allows to use historical process data from multiple processes to generate one or more reliable models based on limited input data from a new process.

The method may further comprise controlling and/or monitoring the process using the spectral model.

After the generation of the spectral model providing process parameter values for a whole duration of an execution of the process, the spectral model may be used by the user for controlling and/or monitoring the process.

The step of using the subset of the stored spectral trajectory data to generate the spectral model providing process parameter values for a substantially whole duration of an execution of the process and the optional subsequent step of controlling and/or monitoring the process using the spectral model, show that past or historical data sets of past or historical processes are used to generate a spectral model, in particular a global generic spectral model. This spectral model may be used to control and/or monitor one or more future or new processes, which are distinct from the past or historical processes.

Accordingly, it is not necessary to run the one or more future or new processes at all in order to receive the process parameter values for the whole duration of these processes. Rather, the use of the spectral model allows for faster and less expensive process development resulting in faster release of a chemical, biopharmaceutical or biotechnological product, and an improved monitoring and/or control of the bioprocess by the user.

Each of the observation data sets may further comprise stored process parameter data and the user-based input data may further comprise input process parameter data.

The user inputs the Raman spectral trajectory data and/or one or more input process descriptors to the database. In addition, the user may input process parameter data. The database algorithm may use the process parameter data given by the user to pre-select a set of spectral trajectories from the database.

The process parameter data may be measured in-line, on-line or at-line by sensors and/or analyzers during process, e.g., pH, glucose, lactate, titer, VCD, intact mass, aggregation, etc. Process parameter data may be set by the user and identified based on the process knowledge of the user. Thus, process parameter data may comprise information about one or more parameters in the process that the user can control or that the user may record, e.g., pH, dissolved oxygen, gas flows, temperature, pressures, or glucose levels.

The process parameter data may comprise critical process parameters (CPP) and/or key process parameters (KPP). A critical process parameter (CPP) may be a process parameter that may have a variability with an impact on a critical quality attribute (CQA) and therefore should be monitored and/or controlled to ensure the process possibly achieves the desired quality. Here, a critical quality attribute is or encompasses a physical, chemical, biological, and/or microbiological property or characteristic that should be within an appropriate limit, range, or distribution to ensure desired product quality. A key process parameter (KPP) may be an adjustable parameter, preferably adjustable in the sense of being variable, of the process that when maintained within a narrow range, possibly ensures optimum process performance. Ranges for key process parameters may be established during process development, and changes to operating ranges may be managed within the quality system. Also, the process parameter data may comprise well-controlled parameters. A well-controlled parameter may be a process parameter which may be controlled by process design and/or standardized procedures or automated control systems to possibly ensure that it remains within the design space of the process. It is only likely to vary beyond the design space if there is a failure in the control system and failure modes for this situation are likely to be mitigated.

Preferably in processes with perfusion bioreactor paired with a large porosity membrane filter, but also in different processes, critical process parameter data may comprise tangential flow filtration (TFF) cross-flow rate, glucose feed rate, nutrient feed rate, antifoam concentration, dissolved oxygen, maximum pCO2, pH, temperature, agitator rate, culture duration, and/or medium osmolality. Key process parameter data may comprise media feed rate, harvest / TFF permeate rate, biomass removal and/or bleed rate, glucose concentration, gas-flow rates, and/or bioreactor weight. Potentially impacted critical quality attributes may comprise antibody-dependent cellular cytotoxicity (ADCC) activity, deaminated isoforms, charge variants, size variants, oligosaccharides such as afucosylated and/or galactosylated glycans, glycosylation related such as sialic acid content, mannose content, nonglycosylated heavy chain, host cell protein (HCP) levels, and/or DNA levels.

Preferably in processes with protein A CMCC, but also in different processes, critical process parameter data may comprise additional considerations such as load UV breakthrough (post-primary load column), load UV flow through (post-secondary load column), start-up / shutdown (initial / final cycles before / after steady state), switch time, protein load ratio (g/L resin), load pH, start elution collection, bioburden reducing solution(s) contact time, and/or impurity wash volume. The key process parameter data may comprise load concentration, end elution collection, column fouling / product cycle count, column bed height, quality of column pack (HETP and asymmetry), and/or flow rates such as loading and/or non-loading. Potentially impacted critical quality attributes may comprise deaminated isoforms, glycosylation related such as sialic acid content, mannose content, and/or non-glycosylated heavy chain, methotrexate, antifoam C, protein A ligand, HCP, DNA, and/or adventitious viral agents (AVA) / viral validation claims.

Preferably in processes with low-pH viral inactivation, but also in different processes, critical process parameter data may comprise mixing model (plug flow reactor) or time / solution homogeneity (chamber / semi-batch), uniformity (plug flow reactor), incubation time, low-pH / acid titrant addition, neutralization pH / base titrant addition, and/or temperature. The key process parameter data may comprise amount of titrant added over time. The potentially impacted critical quality attribute may comprise size exclusion chromatography (SEC) (aggregates / high molecular weight (HMW)), HCP, DNA, adventitious viral agents (AVA) / viral validation claims.

Preferably in processes with anion exchange chromatography, but also in different processes, the critical process parameter data may comprise load concentration, protein load ratio (g/L resin), load conductivity, load pH, product cycle count, column bed height /membrane area, and/or load flow rate. The key process parameter data may comprise start and/or end flow through collection. The potentially impacted critical quality attributes may comprise SEC (aggregates / HMW), protein A ligand, HCP, DNA, and/or adventitious viral agents (AVA) / viral validation claims.

Preferably in processes with polishing bind-and-elute chromatography, but also in other processes, the critical process parameter data may comprise load UV breakthrough (post-primary load column), load UV flow through (post-secondary load column), protein load ratio (g/L resin), start-up / shutdown (initial / final cycles before / after steady state), switch time, start elution collection, end elution collection, load conductivity, load pH, bioburden-reducing solution(s) contact time, and/or impurity wash volume. The key process parameter data may comprise column fouling / product cycle count, load concentration, column bed height, quality of column pack (HETP and/or asymmetry), and/or flow rates such as loading and non-loading. The potentially impacted critical quality attributes may comprise SEC (aggregates / HMW), charge variants, protein A ligand, HCP, DNA, and/or adventitious viral agents (AVA) / viral validation claims.

Preferably in processes with virus filtration, but also in other processes, the critical process parameter data may comprise feed flow rate, load concentration, filtration volume / volumetric loading, load aggregate / particulate level, load conductivity, load pH, process pauses (duration), and/or process pauses (quantity). The key process parameter data may comprise filter usage duration. The potentially impacted critical quality attributes may comprise SEC (aggregates / HMW), and/or adventitious viral agents (AVA) / viral validation claims.

Preferably in processes with single-pass tangential flow filtration, but also in other processes, the critical process parameter data may comprise feed flux, load concentration, membrane mass loading, membrane usage duration, retentate flux, and/or trans-membrane pressure (TMP). Particularly, there may be none key process parameter data and the potentially impacted critical quality attributes may comprise drug substance protein concentration and/or SEC (aggregates / HMW).

Preferably in processes with in-line diafiltration, but also in other processes, the critical process parameter data may comprise buffer feed flow rate, membrane mass loading, membrane usage duration, number of diavolumes, permeate flux, product concentration, product feed flow rate, and/or TMP. Particularly, there may be none key process parameter data and the potentially impacted critical quality attributes may comprise drug substance, protein concentration, SEC (aggregates / HMW), drug substance osmolality, and/or drug substance pH.

Preferably in processes with normal flow/bioburden reduction filtration of the high-concentration ultrafiltration product, but also in other processes, the critical process parameter data may comprise extended filter operating times (days / weeks instead of hours), and/or filter usage duration. The key process parameter data may comprise volumetric loading. The potentially impacted critical quality attributes may comprise drug substance bioburden.

As outlined above, the step of determining a subset of the stored spectral data in accordance with at least one selection criterium by querying the database using the user-input data can be done by using the spectral trajectory data and/or the one or more process descriptors.

In addition, the step of determining a subset of the stored spectral data in accordance with at least one selection criterium by querying the database using the user-input data may be done by using the process parameter data.

The process parameter data might be used as a pre-selection criterium. For example, based on the process parameter data particular observation data sets might be pre-selected, so that the spectral trajectory data are used from these pre-selected observational data sets. The process parameter data might be used in addition to the spectral trajectory data and one or more process descriptors. This ensures an efficient method that selects data in a time-consuming manner in order to generate a more robust spectral model. This model allows for faster and less expensive process development resulting in faster release of a chemical, biopharmaceutical or biotechnological product, and an improved monitoring and/or control of the bioprocess by the user.

The method may further comprise defining the at least one selection criterium in relation to a similarity between the stored observation data sets and the user-based input data, wherein, optionally, defining the selection criterium includes a combination of at least one of the following steps (i) to (iii):
(i) matching of the user-based input data with the stored observation data sets;
(ii) spectral pre-processing (applied to the spectral data of the database (202) and to the provided user-based spectral data), by application of at least one of the following methods:
   - AsLS correction (Asymmetric Least Square Correction, a non-linear baseline is calculated for each spectrum using symmetric least squares algorithm and then baseline is subtracted from the spectrum),
   - moving window (removes noise by applying moving average, or median, window to the spectrum),
   - derivatives,
   - SNV filter (Standard Normal Variate, each observation (spectra) is "normalized" by subtracting the mean and dividing with the standard deviation),
   - MSC (Multiplicative Signal Correction, each spectrum is normalized by regressing it against average spectrum over a selected set of spectra),
   - averaging of the (processed) signal over selected signal range,
   - peak area, peak height, or water band normalization,
   - Multivariate Curve Resolution (MCR);
(iii) comparison of full or parts/phases of process trajectories, by application of at least one of the following methods:
   - Comparison of spectral similarities for specific time points or time intervals in the process,
   - Dynamic Time Warping,
   - Model Arrays,
   - Two one-sided test (TOST) test for time series analysis,
   - (Spectral) similarity index calculation for the whole process,
   - Mechanistic modelling to compare metabolic behavior of processes,
   - Batch Modelling - comparison of batch time trajectories using PLS models;
with the at least one selection criterium being defined by at least one of the following algorithms:
- maximizing correlation coefficient and minimizing averaged distance for each time point in the trajectory of the Batch Evolution Model,
- minimizing total RMSEP between database batch and user trajectory in the Batch Evolution Model,
- minimizing RMSEP for each time point between database batch and user trajectory in the Batch Evolution Model to identify similarity between individual time points related to process stage, and
- minimizing distance to the center of the Batch Level Model.

The selection criterium may be a 0-1 criterium or a linear criterium.

The choice of the criterium may depend on the applied method. In particular, if it would be defined as 0-1 criterium, then stored spectral trajectory data from the database that give a 1:1 result in view of similarity to the input spectral trajectory data will be included in the subset of the stored spectral trajectory data that is used to generate the final spectral model. Alternatively, if the criterium is a continuous one, i.e., a linear criterium, then there will be some ranges applied, for example based on 95% confidence interval. Accordingly, there might be some tolerance allowed between the stored spectral trajectory data and the input spectral trajectory data, when determining a subsect of the stored spectral trajectory data that is used to generate the final spectral model.

This allows for further increasing the spectral model's robustness by including most similar spectral trajectory data in case of the 0-1 criterium, but also by including spectral trajectory data further away in similarity in the model in case of the linear criterium.

The analytical measurements of process parameter may comprise a product quality attribute, or process performance parameter like glucose, lactate and/or titer levels.

A product quality attribute may be a physical, chemical, biological, or microbiological property or characteristic that should be within an appropriate limit, range, or distribution to ensure desired product quality. Example product quality attributes can be glycosylation, charge variants or aggregation pattern.

For example, in-line analytical tools may be used for monitoring product quality attributes.

Accordingly, further considering a product quality attribute contributes to ensure that manufacturing operations are conducted within set specifications. This contributes to the quality of stored data in the database and in turn to an improved spectral model providing process parameter values for a whole duration of an execution of the process.

According to a further aspect of the invention, a computer program product is provided that comprises computer-readable instructions, which, when loaded and executed on a computer system, cause the computer system to perform operations according to the method described before. Accordingly, any of the features of the method described before and their technical effects may also apply to the computer program product of the further aspect of the invention.

According to yet another aspect of the invention, a computer system is provided that is operable to control and/or monitor a process to produce a chemical, biopharmaceutical, or biotechnological product, the computer system comprising a database and one or more processors associated with the database, wherein the database is configured to:
store plural observation data sets associated with respective observations of chemical, biopharmaceutical, or biotechnological past processes, each of the observation data sets comprising stored spectral trajectory data, one or more stored process descriptors, and at least one corresponding actual analytical measurement of a process parameter recorded during performance of a respective past process; wherein the one or more processors are configured to:
obtain, from a user, user-based input data comprising at least one of input spectral trajectory data and one or more input process descriptors;
determine a subset of the stored spectral trajectory data in accordance with at least one selection criterium by querying the database using the user-based input data; and
use the subset of the stored spectral trajectory data to generate a spectral model providing process parameter values for a whole duration of an execution of the process.

In particular, all features of the method and computer program product in accordance with the previous aspects and their respective effects described above might also apply to the computer system.

The one or more processors may be configured to control and/or monitor the process using the spectral model. Each of the observation data sets may further comprise stored process parameter data. The user-based input data from the user may further comprise input process parameter data.

The one or more processors may further be configured to define the at least one selection criterium in relation to a similarity between the stored observation data sets and the user-based input data, wherein, optionally, the one or more processors are configured to define the selection criterium including a combination of at least one of the following steps (i) to (iii):
(i) matching of the user-based input data with the stored observation data sets;
(ii) spectral pre-processing (applied to the spectral data of the database (202) and to the provided user-based spectral data), by application of at least one of the following methods:
   - AsLS correction (Asymmetric Least Square Correction, a non-linear baseline is calculated for each spectrum using symmetric least squares algorithm and then baseline is subtracted from the spectrum),
   - moving window (removes noise by applying moving average, or median, window to the spectrum),
   - derivatives,
   - SNV filter (Standard Normal Variate, each observation (spectra) is "normalized" by subtracting the mean and dividing with the standard deviation),
   - MSC (Multiplicative Signal Correction, each spectrum is normalized by regressing it against average spectrum over a selected set of spectra),
   - averaging of the (processed) signal over selected signal range,
   - peak area, peak height, or water band normalization,
   - Multivariate Curve Resolution (MCR);
(iii) comparison of full or parts/phases of process trajectories (by application of at least one of the following methods:
   - Comparison of spectral similarities for specific time points or time intervals in the process,
   - Dynamic Time Warping,
   - Model Arrays,
   - TOST test for time series analysis,
   - (Spectral) similarity index calculation for the whole process,
   - Mechanistic modelling to compare metabolic behavior of processes,
   - Batch Modelling - comparison of batch time trajectories using PLS models;
with the at least one selection criterium being defined by at least one of the following algorithms:
- maximizing correlation coefficient and minimizing averaged distance for each time point in the trajectory of the Batch Evolution Model,
- minimizing total RMSEP between database batch and user trajectory in the Batch Evolution Model,
- minimizing RMSEP for each time point between database batch and user trajectory in the Batch Evolution Model to identify similarity between individual time points related to process stage, and
- minimizing distance to the center of the Batch Level Model.

The selection criterium may be a 0-1 criterium or a linear criterium. The analytical measurements may comprise a product quality attribute, or process performance parameter as glucose, lactate and/or titer.

Further aspects, features and embodiments of the present disclosure will be described, by way of example, in conjunction with the following drawings.
- Fig. 1: shows a computer-implemented method for generating a spectral model for controlling and/or monitoring a process to produce a chemical, biopharmaceutical, or biotechnological product, according to the aspect,
- Fig. 2: shows a more detailed general workflow of a computer-implemented method for generating a spectral model for controlling and/or monitoring a process to produce a chemical, biopharmaceutical, or biotechnological product, according to the aspect,
- Fig. 3: shows example spectral trajectory data stored in the database or provided by the user as input spectral trajectory data.,
- Fig. 4: shows a computer system operable to control and monitor and/or predict a process to produce a chemical, biopharmaceutical, or biotechnological product, according to the aspect,
- Fig. 5: shows a computer-implemented method for generating a spectral model for controlling and/or monitoring a process to produce a chemical, biopharmaceutical, or biotechnological product, according to a first embodiment of the aspect.

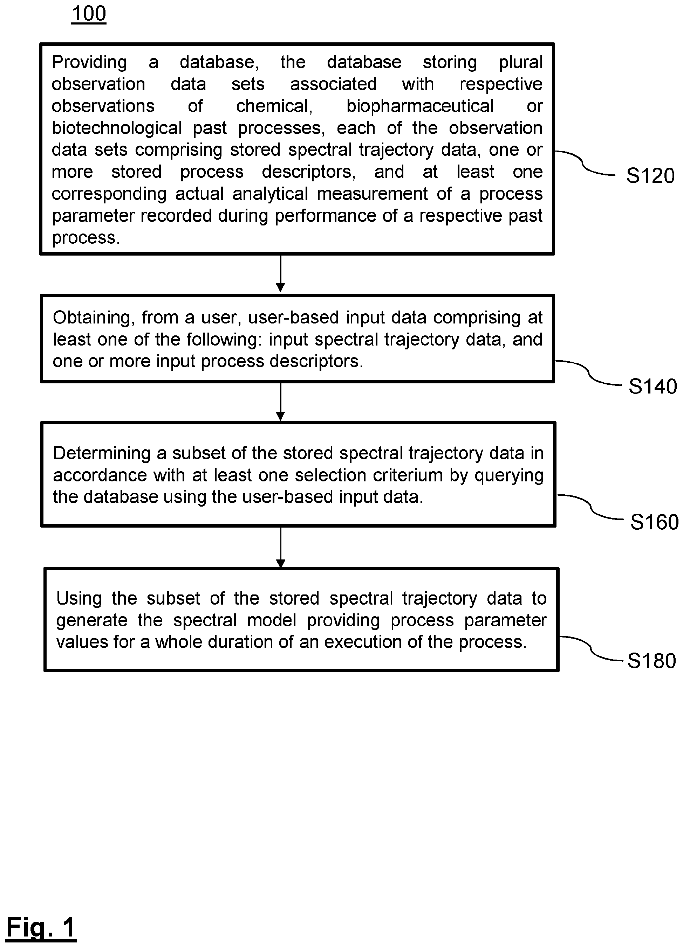

Fig. 1 shows an exemplary workflow of a computer-implemented method 100 for generating a spectral model for controlling and/or monitoring a process, e.g., a process by a spectroscopy system such as a Raman spectroscopy system, to produce a chemical, biopharmaceutical, or biotechnological product, according to the aspect.

The method 100 comprises step S120 of providing a database 202, the database 202 storing plural observation data sets associated with respective observations of chemical, biopharmaceutical, or biotechnological past processes, each of the observation data sets comprising stored spectral trajectory data, one or more stored process descriptors, and at least one corresponding actual analytical measurement of a process parameter recorded during performance of a respective past process.

The method 100 further comprises the step S140 of obtaining, from at least one user, user-based input data comprising at least one of input spectral trajectory data and one or more input process descriptors .

As a next step, the method 100 comprises the step S160 of determining a subset of the stored spectral trajectory data in accordance with at least one selection criterium by querying the database 202 using the user-based input data.

The method 100 also comprises the step S180 of using the subset of the stored spectral trajectory data to generate the spectral model providing process parameter values for a whole duration of an execution of the process.

The method 100 may comprise the step S200 of controlling and/or monitoring the process using the spectral model.

Each of the observation data sets may further comprise stored process parameter data, the user-based input data may further comprise input process parameter data.

The method 100 may comprise defining the at least one selection criterium in relation to a similarity between the stored observation data sets and the user-based input data, wherein, optionally, defining the selection criterium includes a combination of at least one of the following steps: matching of the user-based input data with the stored observation data sets, spectral pre-processing applied to the spectral data of the database 202 and to the provided user-based spectral data, spectral similarity evaluation, and comparison of full or parts/phases of process trajectories. The selection criterium may be a 0-1 criterium or a linear criterium. The analytical measurements of a process parameter may comprise a product quality attribute or process performance parameter as glucose, lactate and/or titer.

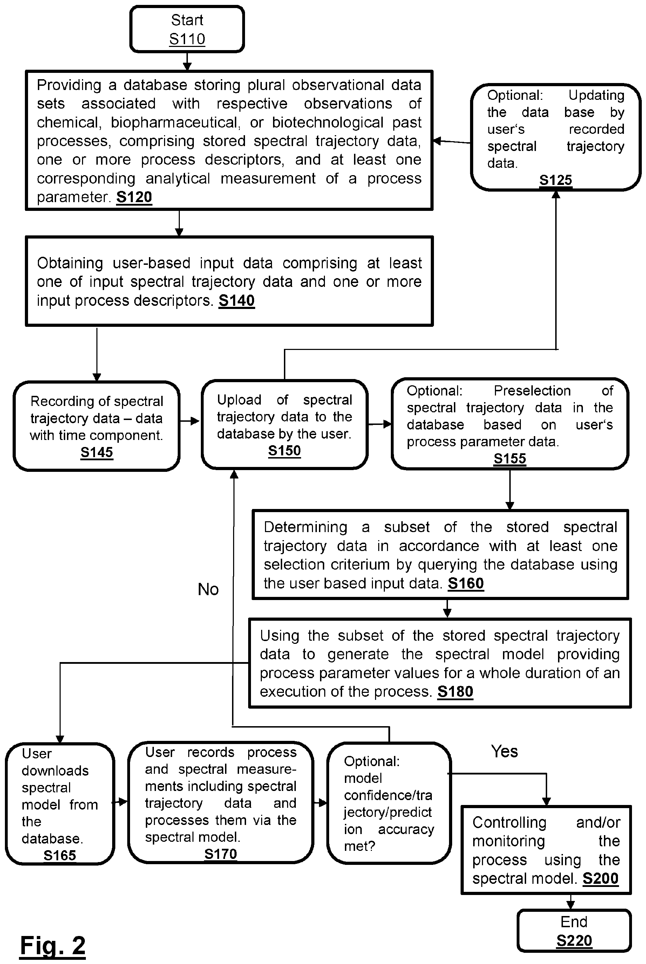

Fig. 2 shows a general workflow of the computer-implemented method 100 for generating a spectral model for controlling and/or monitoring a process to produce a chemical, biopharmaceutical, or biotechnological product, according to the aspect.

The method 100 comprises the previously described steps: S120 of providing a database, S140 of obtaining user-based input data, S160 of determining a subset of the stored spectral trajectory data, and S180 of using the subset of the stored spectral trajectory data to generate the spectral model.

In particular, the method 100 may begin at a step S120.

In step S120 of providing a database, the database 202 is generated by storing plural observational data sets associated with respective observations of chemical, biopharmaceutical, or biotechnological past processes, comprising stored spectral trajectory data, one or more process descriptors, and at least one corresponding analytical measurement of a process parameter.

The step S120 of providing a database may be followed by process documentation by the user that in turn allows for step S140, i.e., obtaining user-based input data comprising at least one of input spectral trajectory data and one or more input process descriptors, that may include step S145, i.e., recording of spectral trajectory data, i.e., data with time component, and/or step S150, i.e., uploading of spectral trajectory data to the database 202 by the user, and optionally, updating S125 the database 202 by the users recorded spectral trajectory data, and/or step S155, i.e., preselecting of spectral trajectory data in the database 202 based on the user's process parameter data .

According to a different concept, there is disclosed a method that comprises a step of providing a database that comprises storing plural observational data sets associated with respective observations of chemical, biopharmaceutical, or biotechnological past processes, comprising stored spectral data without time component, one or more process descriptors, and at least one corresponding analytical measurement of a process parameter. In this different concept, the step of providing a database may be followed by process documentation by the user that in turn allows for a step of obtaining user-based input data comprising at least one of input spectral data without time component and one or more input process descriptors, that may include the steps of recording of spectral data without time component, and/or uploading of spectral data without time component to the database by the user, and optionally, updating the database by the users recorded spectral data without time component, and/or preselecting of spectral data in the database based on the user's process descriptor data. Accordingly, in the different concept, data that is compared with each other is not time dependent. In particular, the user-based input data that is to be compared with the data stored in the database may be based on a medium before the biopharmaceutical process starts or a search spectrum at a given point of time of a biopharmaceutical process that is used as input for the database.

According to the aspect of the invention, and as shown in Fig. 2, the method 100 proceeds with step S160 of determining a subset of the stored spectral trajectory data in accordance with at least one selection criterium by querying the database using the user-based input data, that is followed by step S180 of using the subset of the stored spectral trajectory data to generate the spectral model providing process parameter values for a whole duration of an execution of the process. Accordingly, a spectral calibration model such as a Raman-based monitoring model may be obtained. Such a calibration model may be a partial least squares (PLS) model, although other approaches can be used as well, e.g., a multiple linear regression model. In particular, partial least squares (PLS) and/or multiple linear regression modeling methods may be used to correlate the Raman spectra to the analytical measurements. To build the calibration model, spectra may be processed with one or a combination of several spectral pre-processing methods, e.g., AsLS, SNV, MSC, derivatives, peak area, peak height, and/or water band normalization. Multivariate Curve Resolution (MCR) can be used to deconvolute signal of known media components like glucose, glutamine from the rest of the spectrum and this way facilitate comparison of deconvoluted spectral 'backgrounds' for improved similarity search. So, the spectra pre-processing can be used both to improve database search, but is also advantageous to obtain good spectral models. To improve the models, only part of the spectrum may be selected, for example only bands corresponding to the Raman signal of the molecule which concentration is to be predicted may be included. Most relevant part of the spectrum for model building can also be obtained with the application of different variable selection methods. Processing of spectra could involve taking a part of a spectrum, and this part can be decided based on process knowledge or applying some variable selection approach. In addition, in model building different scaling can be applied to the data, for example centering may be used for spectral models.

After generating the spectral model, the user may download the spectral model from the database (S165), wherein, optionally, the user may upload the spectral model to a place in the system where it will be used to generate process parameter values. The user may record process and spectral measurements including spectral trajectory data and may process them via the spectral model (S170).

Also, the method may contain the optional step of evaluating whether a model confidence and/or trajectory and/or prediction accuracy is met. In case that the accuracy is met, the method 100 proceeds with step S200 of controlling and/or monitoring the process using the spectral model. In case that the accuracy is not met, the method 100 turns back to step S150 of uploading of spectral trajectory data to the database. However, in case that the method may not contain the optional step of evaluating whether a model confidence and/or trajectory and/or prediction accuracy is met, the step S170, i.e., recording process and spectral measurements including spectral trajectory data and processing them via the spectral model, would be immediately followed by the step S200, i.e., controlling and/or monitoring the process using the spectral model.

With respect to the step of evaluating whether a model confidence and/or trajectory and/or prediction accuracy, it might be possible to use RMSEP values in order to check if the prediction error of the process parameter value is within pre-defined acceptance criteria. Alternatively, or in addition, predictions of the specific process parameter value obtained from the new process with the applied model might be compared with off-line reference values obtained for the same samples from the process. The evaluation might be based on the criterium that in case of the predictions are within pre-defined acceptance criteria, e.g., within a range of approximately +/- 10%, then the model is considered as good.

After step S200, the method may end at step S220.

Any of the steps S110 to S220 described before can be at least partly executed as cloud application and/or as desktop application. The different steps of method 100 as described with respect to Fig. 2 may be conducted in the sequence as indicated by the arrows in Fig. 2. However, an alternative sequence of these process steps may be conceivable.

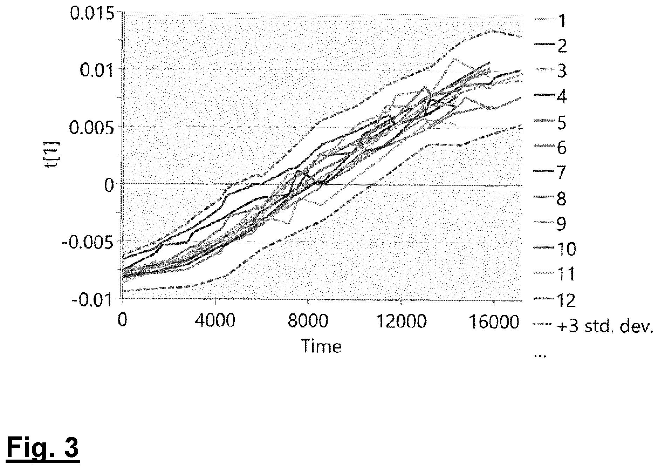

Fig. 3 shows example spectral trajectory data stored in the database or provided by the user as input spectral trajectory data.

The example process trajectories summarize in one component the evolution of the bioprocess as monitored with spectral data with time. Fig. 3 shows twelve different trajectories (see solid lines 1 to 12 in Fig. 3), wherein each of these trajectories may come from a different experiment. The spectral data (Y-axis) is plotted over the time (X-axis). The spectral data labeled "t[1]" in Fig. 3 may refer to the first score of a BEM model, e.g., as a 'summary' of the time spectral trajectory for each of the different experiments. The mean trajectory of these twelve different trajectories as well as three standard deviations around the mean are shown as dashed lines.

When querying the database using the time-dependent input data, the user may provide user-based input spectroscopy batch data of several cultivations, e.g., N cultivations, and/or additional relevant process parameter data. Each of the N cultivations may be represented by a trajectory as shown in Fig. 3.

The input spectroscopy batch data may be data with a time component, i.e., input spectral trajectory data. Accordingly, relevant spectral trajectory data from the database that is used for generating the spectral model may be obtained by comparison of input spectral process trajectories obtained from the user with the spectral process trajectories stored in the database.

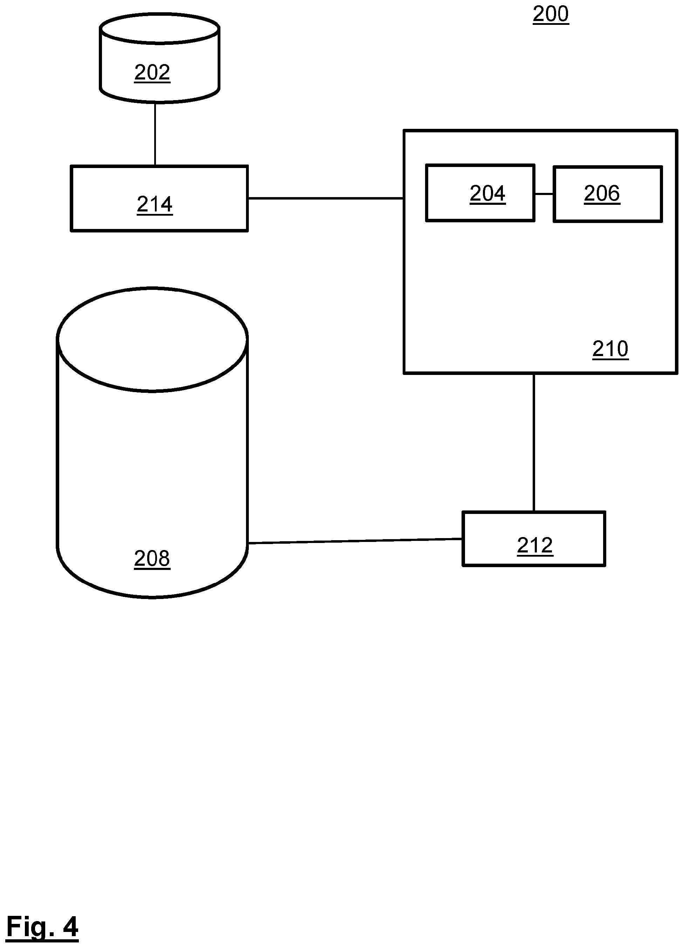

Fig. 4 shows a computer system 200 operable to control and monitor and/or predict a process to produce a chemical, biopharmaceutical, or biotechnological product, according to the aspect. Particularly, the computer system 200 is adapted to perform the computer-implemented method 100 described above.

The computer system 200 comprises the database 202 and one or more processors 204 associated with the database 202. The one or more processors 204 may be part of a control system 210, more specifically a bioprocess control system 210, that further may comprise at least one control device 206 that may be connected and/or communicate with the one or more processors 204.

The database 202 is configured to store plural observation data sets associated with respective observations of chemical, biopharmaceutical, or biotechnological past processes, each of the observation data sets comprising stored spectral trajectory data, one or more stored process descriptors, and at least one corresponding actual analytical measurement of a process parameter recorded during performance of a respective past process. Particularly, the database 202 that is provided in the step S120 described with respect to Figs. 1 and 2 might be the database shown and described with respect to Fig. 4.

The one or more processors 204 perform the steps S140 to S200 described with respect to Figs. 1 and 2. Particularly, the one or more processors 204 are configured to obtain S140, from a user, user-based input data comprising at least one of input spectral trajectory data and one or more input process descriptors. The one or more processors 204 are further configured to determine S160 a subset of the stored spectral data in accordance with at least one selection criterium by querying the database using the user-based input data, and to use S180 the subset of the stored spectral trajectory data to generate a spectral model providing process parameters values for a whole duration of an execution of the process. Furthermore, the one or more processors 204 may be configured to control and/or monitor S200 the process using the spectral model.

The computer system 200 may further comprise and/or may be connected and/or may communicate with a recommendation system 214. The generated spectral model may be transferred to the bioprocess control system 210 from the recommendation system 214 that may be provided by a provider of the bioprocess control system 210. The recommendation system 214 may be connected and/or communicate with the database 202.

The bioprocess control system 210 may communicate via the one or more processors 204 with the recommendation system 214 that in turn may communicate with the database 202, both of which may be provided by a provider of the process control system 210. The recommendation system 214 may be software that is configured to connect to the database 202 and allow the user to query it, e.g., via the control device 206, e.g., a personal computer, which is connected with the one or more processors 204. Furthermore, the recommendation system 214 may be configured to extract relevant stored spectral trajectory data from the database 202, build the spectral model and send the spectral model back to the process control system 210 and/or Raman analyzer 212.

As an equipment for the computer-implemented method for generating a spectral model for controlling and/or monitoring a process to produce a chemical, biopharmaceutical, or biotechnological product, the user may have several components of a technological platform, e.g., the recommendation system 214. These components may either all provided by one provider or may have the necessary communication interfaces to each other. This description is a concrete example, but not a universal equipment. Other equipment may be possible.

The computer system 200 may be connected and/or communicate with a bioreactor 208. For example, the bioreactor 208 may be implemented as a 50L multi-use bioreactor or single-use bioreactor, e.g., a Biostat° STR50 with FlexSafe 50L bag equipped with BioPAT° Spectro Port for Raman measurements (Sartorius Stedim Biotech GmbH). Alternatively, culture vessels with working volumes of 5 L, 10 L, 15 L, 20 L and 30 L may be used.

The bioreactor 208 may include control and/or inline and/or online measurement capability. In particular, the bioreactor 208 may comprise or may be connected with the bioprocess control system. The bioreactor may be connected with the Raman analyzer 212 and/or Raman spectrometer with at least one Raman immersion probe placed in or at the bioreactor 208 to continuously measure a Raman spectrum.

The bioreactor 208 may be capable of controlling and/or measuring one or more of the following: temperature, pH, dissolved oxygen (DO) concentration, or cell density of cells. The bioreactor 208 may be capable of recording a variety of measurements using the measurement devices mentioned above and further measurement devices.

In conjunction with the at least one control device 206, the bioreactor 208 particularly may be capable of performing various forms of inline measurement and/or analysis, in which a medium is measured while remaining in the bioreactor 208.

The at least one control device 206 may be connected and/or communicate and/or comprised by the bioprocess control system 210 that in turn may be connected and/or communicate with a spectrometer control system comprising and/or operating the Raman spectrometer and/or the Raman analyzer 212 and, preferably via the Raman analyzer 212, with the bioreactor 208. The Raman analyzer 212 may be connected and/or communicate with the bioreactor 208.

Accordingly, there may be provided a system controlling a bioprocess, i.e., the bioprocess control system 210, on the local level (e.g. BioBrain° (Sartorius Stedim Biotech GmbH) based control unit). There may be further provided a spectrometer control system for operating the Raman spectrometer and/or the Raman analyzer 212 and which receives spectra. Furthermore, there may be a kind of supervisory control system (e.g. BioPAT° MFCS Scada system), which is connected to the database 202 and an advisory subsystem, e.g., the recommendation system 214, and receives data from the bioprocess control system 210, e.g., a bioprocess controller (Biobrain^{®} (Sartorius Stedim Biotech GmbH)), and spectrometer control system and initiates equipment phases and control loops on the local level.

The Raman analyzer 212 may be configured to record a time-dependent spectrum of a bioprocess processed in the bioreactor 208 and/or may be used by the spectral model as the above-described spectral trajectory data to provide a real-time prediction, e.g., for concentration of the substrate glucose and the metabolite lactate in the bioreactor 208. The real-time prediction for concentration of the substrate glucose and metabolite lactate in the bioreactor 208 may be used to control the mentioned parameters, e.g., via proportional-integral-derivative (PID) feedback loops.

The database 202 can be a relational database in which the data are stored in related tables. The communication can take place for example by means of a database query language (e.g., Structured Query Language SQL). The database 202 may be hosted on a server system and/or may be accessible to users via web access, e.g., a cloud. The database 202 may reside on a server cluster which is under the control of a provider.

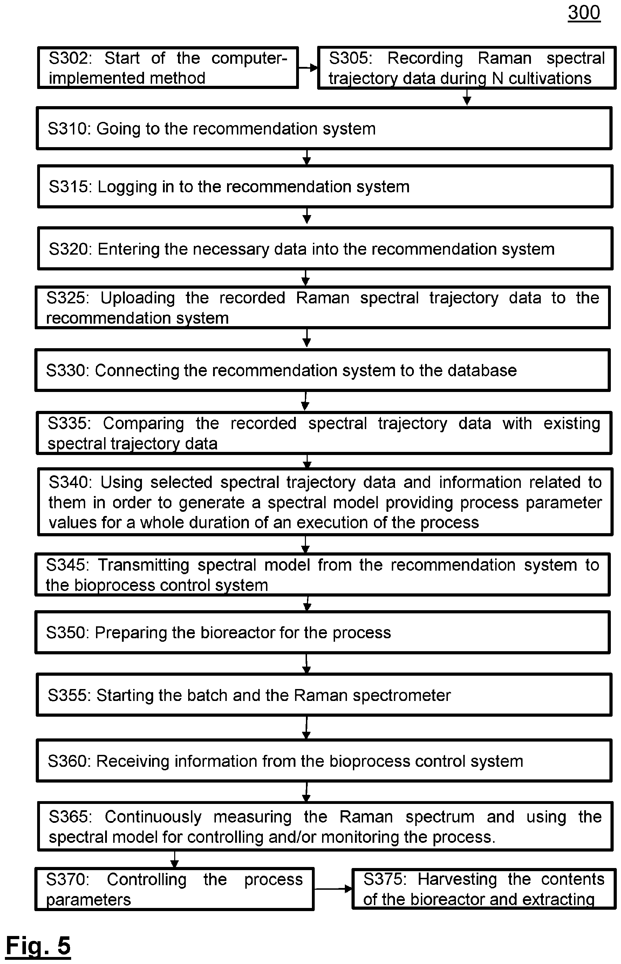

Fig. 5 shows a computer-implemented method 300 for generating a spectral model for controlling and/or monitoring a process to produce a chemical, biopharmaceutical, or biotechnological product, according to a first embodiment of the aspect.

In the embodiment shown in Fig. 5, monitoring and/or control of a biopharmaceutical process is exemplarily described.

The aim of the method 300 is to obtain a spectral (calibration) model to monitor and/or control process parameters and/or monitor product concentration and quality, depending on the cell line, the medium, the product and process parameters. The user knows what the user wants to produce, and the user may have the platform technology to achieve this goal. However, the user may not have any or has limited number of time-dependent spectral data, i.e., spectral trajectory data, for generation of the spectral model. However, method 300 may be used to generate the spectral model.

The present disclosure will be exemplarily described with respect to a monoclonal antibody (mAb) in a CHO cell line in the bioreactor 208, the present disclosure is also suitable for all other biopharmaceutical processes for the production of biopharmaceutical products (i.e., recombinant and non-recombinant proteins; vaccines; gene vectors; DNA; RNA; antibiotics, secondary metabolites, growth factors, cells for cell therapy or regenerative medicine; semi-synthetic products such as artificial organs, etc.) in manufacturing systems like cell-based systems such as animal cells (e.g. CHO, HEK, PerC6, VERO, MDCK, etc.); insect cells (e.g. SF9, SF21), microorganisms (e.g. E. coli, S. cerevisiae, P. pastoris, etc.), algae; plant cells; cell-free expression systems (cell extracts, recombinant ribosomal systems, etc.); primary cells; stem cells; native and genetically modified cells; matrix-based cell systems, etc.

The present disclosure provides a significant user benefit not only for the bioreactor processes mentioned with respect to the first embodiment shown in Fig. 5, but for all production stages in which at least one process-critical process parameter (CPP), meaning a parameter that influences the process flow (Process Key Performance Indicator - Process KPI) and/or product titer, yield or quality (Critical Quality Attribute - CQA) is measured and/or controlled.

The method 300 may begin at step S302.

At step S305, the user may record Raman spectral trajectory data during one or more cultivations. In addition, the user may record input process parameter data. Accordingly, time dependent trajectory data are sampled during performance of a process. This distinguishes the method 300 from the different concept that uses data that is not time dependent, wherein the user records a Raman spectrum at a particular point of time, e.g., a particular Raman spectrum of a cell medium before inoculation.

At step S310, the user may go or switch to the recommendation system 214.

At step S315, the user may log in to the recommendation system 214 with his/her username, if required.

At step S320, the user may enter necessary data characterizing the process into the recommendation system 214. Examples of the necessary data characterizing the process are one or more of the following:
a. Biological type of the process: CHO-based
b. Scaling of the process: 10L
c. Type of the process
   i. Type of product: mAb
   ii. Type of process: Fed batch
d. Biological system
   i. Cellca 2 line
e. Media platform
   i. Smart CHO
f. Critical Quality Attributes (CQA) to monitor
   i. Product concentration
   ii. Glycan profile
g. Critical process parameters (CPP) to monitor
   i. Glucose concentration
   ii. Lactate concentration

In particular, the user may enter at least one of these necessary data into the recommendation system 214. The data under points a to e are those parameters that might be used for pre-selection of relevant spectral trajectory data from the database and are not all mandatory. The data under points f and g might be entered so that the system knows what model should be generated and are mandatory in order to extract spectra that have the relevant CQA or CPP values connected to them.

At step S325, the user may upload the at least one Raman spectral trajectory data recorded in step 305 to the recommendation system 214.

The method 300 may further comprise the previously described steps S120, S140, S160, and/or S180, i.e.,
- providing S120 a database 202, the database 202 storing plural observation data sets associated with respective observations of chemical, biopharmaceutical, or biotechnological past processes, each of the observation data sets comprising stored spectral trajectory data, one or more stored process descriptors, and at least one corresponding actual analytical measurement of a process parameter recorded during performance of a respective past process,
- obtaining S140, from a user, user-based input data comprising at least one of input spectral trajectory data and one or more input process descriptors,
- determining S160 a subset of the stored spectral trajectory data in accordance with at least one selection criterium by querying the database 202 using the user-based input data, and/or
- using S180 the subset of the stored spectral trajectory data to generate the spectral model providing process parameter values for a whole duration of an execution of the process.

Particularly, performing the steps S120, S140, S160, and S180, may comprise performing the steps S330 to S340 as follows:
At step S330, and after completion of the data entry, the recommendation system 214 may connect to the database 202 that stores Raman spectral trajectory data, one or more process descriptors, and at least one corresponding actual analytical measurement of a process parameter recorded during performance of a respective past process. With this respect, the database 202 stores one or more reference data, e.g., product concentration, glucose concentration, lactate concentration, etc.

At step S335, the recorded spectral trajectory data may be compared with plural existing spectral trajectory data (particularly with the totality of the existing spectra) in the database 202. The comparison may comprise:
a. From all available spectra Raman spectra may be selected based on the one or more input parameters, e.g., Raman spectra from a CHO process, 10L fed batch scale, mAb product, Cellca 2 line, Smart CHO medium exemplarily are selected.
b. Selected spectra together with the relevant data connected to them: product, glucose, lactate concentration value and/or glycan profile are transferred to the recommendation system 214.
c. Transferred spectra may be compared by the recommendation system 214 with the input spectrum particularly as follows:
   i. At least one spectra pre-processing method may be tested on the input and transferred spectra and general similarity between non-processed and processed spectra may be measured or determined with at least one spectral similarity measure algorithm. The optimal pre-processing/similarity algorithm combination may be selected based on the closest similarity between input and transferred spectra.
   ii. For the selected pre-processing/similarity algorithm combination the algorithm may select most similar spectra that may at the same time cover the maximum concentration range for given critical process parameters (CPPs) or critical quality attributes (CQA).

At step S340, the recommendation system 214 may use selected spectral trajectory data and information related to them in order to generate a spectral model providing process parameter values for a whole duration of an execution of the process. The related information may be product, glucose, lactate concentration or glycan profile to automatically build a partial least square (PLS) and/or orthogonal partial least square OPLS° calibration model(s) - in short (O)PLS - for prediction of product, glucose, lactate concentration or glycan profile. Automated model building procedure may include application of spectra pre-processing step(s) selected in step S335.

The method 300 may further comprise the previously described step S200, i.e., controlling and/or monitoring the process using the spectral model. Particularly, performing the step S200 may comprise performing the steps S345 to S375 as follows:
At step S345, spectral models from the recommendation system 214 may be transmitted to the bioprocess control system 210. For example, (O)PLS Raman calibration model for product concentration, (O)PLS Raman calibration model for glucose concentration, (O)PLS Raman calibration model for lactate concentration, and/or (O)PLS Raman calibration model for amount of specific glycan species, may be transmitted from the recommendation system 214 to the bioprocess control system 210.

The bioprocess control system 210 now has the necessary information so that the batch may be started and the process may be controlled and/or monitored.

At step S350, the user may prepare the bioreactor 208 for the process by filling the reactor sterile with medium and making necessary connections.

At step S355, the user may start the batch and/or the Raman spectrometer.

As soon as all set points in the bioreactor 208 are reached, the user may receive information from the bioprocess control system 210 that the bioreactor 208 can be incubated with the Cellca 2 cell line. This may be done by the user at step S360. Alternatively, incubation with the Cellca 2 cell line may be initiated in an automated way.

At step S365, the Raman spectrum may be continuously measured using a Raman probe connected to the Raman spectrometer that sends Raman spectra to the Raman calibration model(s) implemented in the bioprocess control system 210.

At step S370, the bioprocess control system 210 may control the process parameter(s), e.g., lactate and/or glucose concentration, and/or may monitor CQAs (product concertation and/or glycosylation profile) via the output from the Raman calibration models. Additional at-line or off-line measurements for glucose, lactate, product concentration and/or glycosylation may be taken once per day. Once the recipe has gone through completely, the batch is considered complete.

At step S375, the user may harvest the contents of the bioreactor 208 and may extract from it the monoclonal antibody (mAb). The process control system may then transfer the Raman spectra with the available at-line or off-line data for glucose, lactate, product concentration and/or glycosylation via the recommendation system 214 to the database 202. In the database 202, the number of available data has increased, and may be made available to other users.

The different steps of method 300 as described with respect to Fig. 5 may be conducted in the sequence as described before and indicated by the arrows in Fig. 5. However, an alternative sequence of these process steps may be conceivable.

### List of Reference Numerals

- 100: Computer-implemented method
- S110: Start of the computer-implemented method
- S120: Step of providing a database
- S125: Step of updating the database
- S140: Step of obtaining user-based input data
- S145: Step of recording of spectral trajectory data
- S150: Step of uploading of spectral trajectory data to the database
- S155: Step of preselecting of spectral trajectory data in the database
- S160: Step of determining a subset of stored spectral trajectory data
- S165: Step of downloading calibration model from the database
- S170: Step of recording process and spectral measurements including spectral trajectory data and processing them via the spectral model
- S180: Step of using the subset of stored trajectory data to generate the spectral model
- S200: Controlling and/or monitoring the process using the spectral model
- S220: End of the computer-implemented method
- 200: Computer system
- 202: Database
- 204: One or more processors
- 206: At least one control device
- 208: Bioreactor
- 210: Bioprocess control system
- 212: Raman analyzer
- 214: Recommendation system
- 300: Computer-implemented method according to a first embodiment
- S302: Start of the computer-implemented method
- S305: Step of recording Raman spectral trajectory data during N cultivations
- S310: Step of going to the recommendation system 214
- S315: Step of logging in to the recommendation system 214
- S320: Step of entering the necessary data characterizing the process into the recommendation system 214
- S325: Step of uploading the Raman spectral trajectory data recorded in step 305 to the recommendation system 214
- S330: Step of connecting the recommendation system 214 to the database 202
- S335: Step of comparing the recorded spectral trajectory data with the totality of the existing spectral trajectory data in the database 202
- S340: Step of using selected spectral trajectory data and information related to them in order to generate a spectral model
- S345: Step of transmitting spectral model from the recommendation system 214 to the bioprocess control system 210
- S350: Step of preparing the bioreactor 208 for the process by filling the reactor sterile with medium and making all necessary connections
- S355: Step of starting the batch and the Raman spectrometer
- S360: Step of receiving information from the bioprocess control system 210 that the bioreactor 208 can be incubated with the Cellca 2 cell line
- S365: Step of continuously measuring the Raman spectrum using a Raman probe connected to the Raman spectrometer
- S370: Step of controlling the process parameters by the bioprocess control system 210
- S375: Step of harvesting the contents of the bioreactor 208 and extracting from it the monoclonal antibody (mAb)

## Claims

1. A computer-implemented method (100) for generating a spectral model for controlling and/or monitoring a process to produce a chemical, biopharmaceutical, or biotechnological product, comprising:
- providing (S120) a database (202), the database (202) storing plural observation data sets associated with respective observations of chemical, biopharmaceutical, or biotechnological past processes, each of the observation data sets comprising:
- stored spectral trajectory data,
-- one or more stored process descriptors, and
-- at least one corresponding actual analytical measurement of a process parameter recorded during performance of a respective past process;
- obtaining (S140), from a user, user-based input data comprising at least one of the following:
-- input spectral trajectory data, and
-- one or more input process descriptors;
- determining (S160) a subset of the stored spectral trajectory data in accordance with at least one selection criterium by querying the database (202) using the user-based input data; and
- using (S180) the subset of the stored spectral trajectory data to generate the spectral model providing process parameter values for a whole duration of an execution of the process.

2. The method of claim 1, further comprising controlling and/or monitoring (S200) the process using the spectral model.

3. The method of claim 1 or 2, further comprising:
defining the at least one selection criterium in relation to a similarity between the stored observation data sets and the user-based input data, wherein, optionally, defining the selection criterium includes a combination of at least one of the following steps (i) to (iii):
(i) matching of the user-based input data with the stored observation data sets;
(ii) spectral pre-processing (applied to the spectral data of the database (202) and to the provided user-based spectral data), by application of at least one of the following methods:
- AsLS correction (Asymmetric Least Square Correction, a non-linear baseline is calculated for each spectrum using symmetric least squares algorithm and then baseline is subtracted from the spectrum),
- moving window (removes noise by applying moving average, or median, window to the spectrum),
- derivatives,
- SNV filter (Standard Normal Variate, each observation (spectra) is "normalized" by subtracting the mean and dividing with the standard deviation),
- MSC (Multiplicative Signal Correction, each spectrum is normalized by regressing it against average spectrum over a selected set of spectra),
- averaging of the (processed) signal over selected signal range,
- peak area, peak height, or water band normalization,
- Multivariate Curve Resolution (MCR);
(iii) comparison of full or parts/phases of process trajectories, by application of at least one of the following methods:
- Comparison of spectral similarities for specific time points or time intervals in the process,
- Dynamic Time Warping,
- Model Arrays,
- TOST test for time series analysis,
- (Spectral) similarity index calculation for the whole process,
- Mechanistic modelling to compare metabolic behavior of processes,
- Batch Modelling - comparison of batch time trajectories using PLS models;
with the at least one selection criterium being defined by at least one of the following algorithms:
- maximizing correlation coefficient and minimizing averaged distance for each time point in the trajectory of the Batch Evolution Model,
- minimizing total RMSEP between database batch and user trajectory in the Batch Evolution Model,
- minimizing RMSEP for each time point between database batch and user trajectory in the Batch Evolution Model to identify similarity between individual time points related to process stage, and
- minimizing distance to the center of the Batch Level Model.

4. The method of any one of the preceding claims, wherein the selection criterium is a 0-1 criterium or a linear criterium.

5. The method of any one of the preceding claims, wherein the analytical measurements of process parameter comprise a product quality attribute, or process performance parameter like glucose, lactate and/or titer.

6. A computer program product comprising computer-readable instructions, which, when loaded and executed on a computer system, cause the computer system to perform operations according to the method (100) of any one of the preceding claims.

7. A computer system (200) operable to control and/or monitor a process to produce a chemical, biopharmaceutical, or biotechnological product, the computer system (200) comprising a database (202) and one or more processors (204) associated with the database (202),
wherein the database (202) is configured to:
- store (S120) plural observation data sets associated with respective observations of chemical, biopharmaceutical, or biotechnological past processes, each of the observation data sets comprising:
- stored spectral trajectory data,
-- stored process descriptors, and
-- at least one corresponding actual analytical measurement of a process parameter recorded during performance of a respective past process;
wherein the one or more processors (204) are configured to:
- obtain (S140), from a user, user-based input data comprising at least one of the following:
-- input spectral trajectory data, and
-- input process descriptors;
- determine (S160) a subset of the stored spectral trajectory data in accordance with at least one selection criterium by querying the database (202) using the user-based input data; and
- use (S180) the subset of the stored spectral trajectory data to generate a spectral model providing process parameter values for a whole duration of an execution of the process.

8. The method of claim 7, wherein the one or more processors (204) are configured to control and/or monitor (S200) the process using the spectral model.

9. The computer system of claim 7 or 8, wherein the one or more processors (204) are further configured to:
define the at least one selection criterium in relation to a similarity between the stored observation data sets and the user-based input data, wherein, optionally, the one or more processors (204) are configured to define the selection criterium including a combination of at least one of the following steps (i) to (iii):
(i) matching of the user-based input data with the stored observation data sets;
(ii) spectral pre-processing (applied to the spectral data of the database (202) and to the provided user-based spectral data), by application of at least one of the following methods:
- AsLS correction (Asymmetric Least Square Correction, a non-linear baseline is calculated for each spectrum using symmetric least squares algorithm and then baseline is subtracted from the spectrum),
- moving window (removes noise by applying moving average, or median, window to the spectrum),
- derivatives,
- SNV filter (Standard Normal Variate, each observation (spectra) is "normalized" by subtracting the mean and dividing with the standard deviation),
- MSC (Multiplicative Signal Correction, each spectrum is normalized by regressing it against average spectrum over a selected set of spectra),
- averaging of the (processed) signal over selected signal range,
- peak area, peak height, or water band normalization,
- Multivariate Curve Resolution (MCR);
(iii) comparison of full or parts/phases of process trajectories (by application of at least one of the following methods:
- Comparison of spectral similarities for specific time points or time intervals in the process,
- Dynamic Time Warping,
- Model Arrays,
- TOST test for time series analysis,
- (Spectral) similarity index calculation for the whole process,
- Mechanistic modelling to compare metabolic behavior of processes,
- Batch Modelling - comparison of batch time trajectories using PLS models;
with the at least one selection criterium being defined by at least one of the following algorithms:
- maximizing correlation coefficient and minimizing averaged distance for each time point in the trajectory of the Batch Evolution Model,
- minimizing total RMSEP between database batch and user trajectory in the Batch Evolution Model,
- minimizing RMSEP for each time point between database batch and user trajectory in the Batch Evolution Model to identify similarity between individual time points related to process stage, and
- minimizing distance to the center of the Batch Level Model.

10. The computer system of any one of claims 7 to 9, wherein the selection criterium is a 0-1 criterium or a linear criterium.

11. The computer system of any one of claims 7 to 10, wherein the analytical measurements of process parameter comprise a product quality attribute, or process performance parameter like glucose, lactate and/or titer.

## Amended claims

### Amended claims in accordance with Rule 137(2) EPC.

1. A computer-implemented method (100) for generating a spectral model for controlling and/or monitoring a process to produce a chemical, biopharmaceutical, or biotechnological product, comprising:
- providing (S120) a database (202), the database (202) storing plural observation data sets associated with respective observations of chemical, biopharmaceutical, or biotechnological past processes, each of the observation data sets comprising:
-- stored spectral trajectory data,
-- one or more stored process descriptors, and
-- at least one corresponding actual analytical measurement of a process parameter recorded during performance of a respective past process;
- obtaining (S140), from a user, user-based input data comprising at least one of the following:
-- input spectral trajectory data, and
-- one or more input process descriptors;
- determining (S160) a subset of the stored spectral trajectory data in accordance with at least one selection criterium by querying the database (202) using the user-based input data; and
- using (S180) the subset of the stored spectral trajectory data to generate the spectral model providing process parameter values for a whole duration of an execution of the process.

2. The method of claim 1, wherein the stored spectral trajectory data comprises spectral data or spectra with associated time information, and
wherein the input spectral trajectory data comprises spectral data or spectra with associated time information.

3. The method of claim 1, further comprising controlling and/or monitoring (S200) the process using the spectral model.

4. The method of claims 1 to 3, further comprising:
defining the at least one selection criterium based on a similarity between the stored observation data sets and the user-based input data.

5. The method of claim 4, wherein defining the selection criterium includes at least one of the following steps (i) to (iii):
(i) matching of the user-based input data with the stored observation data sets;
(ii) spectral pre-processing (applied to the spectral data of the database (202) and to the provided user-based spectral data), by application of at least one of the following methods:
- AsLS correction (Asymmetric Least Square Correction, a non-linear baseline is calculated for each spectrum using symmetric least squares algorithm and then baseline is subtracted from the spectrum),
- moving window (removes noise by applying moving average, or median, window to the spectrum),
- derivatives,
- SNV filter (Standard Normal Variate, each observation (spectra) is "normalized" by subtracting the mean and dividing with the standard deviation),
- MSC (Multiplicative Signal Correction, each spectrum is normalized by regressing it against average spectrum over a selected set of spectra),
- averaging of the (processed) signal over selected signal range,
- peak area, peak height, or water band normalization,
- Multivariate Curve Resolution (MCR);
(iii) comparison of full or parts/phases of process trajectories, by application of at least one of the following methods:
- Comparison of spectral similarities for specific time points or time intervals in the process,
- Dynamic Time Warping,
- Model Arrays,
- TOST test for time series analysis,
- (Spectral) similarity index calculation for the whole process,
- Mechanistic modelling to compare metabolic behavior of processes,
- Batch Modelling - comparison of batch time trajectories using PLS models;
wherein the at least one selection criterium is defined by at least one of the following algorithms:
- maximizing correlation coefficient and minimizing averaged distance for each time point in the trajectory of the Batch Evolution Model,
- minimizing total RMSEP between database batch and user trajectory in the Batch Evolution Model,
- minimizing RMSEP for each time point between database batch and user trajectory in the Batch Evolution Model to identify similarity between individual time points related to process stage, and
- minimizing distance to the center of the Batch Level Model.

6. The method of any one of the preceding claims, wherein the selection criterium is a 0-1 criterium or a linear criterium.

7. The method of any one of the preceding claims, wherein the analytical measurements of process parameter comprise a product quality attribute, or concentrations of substrates like glucose, lactate and/or titer.

8. A computer program product comprising computer-readable instructions, which, when loaded and executed on a computer system, cause the computer system to perform operations according to the method (100) of any one of the preceding claims.

9. A computer system (200) operable to control and/or monitor a process to produce a chemical, biopharmaceutical, or biotechnological product, the computer system (200) comprising a database (202) and one or more processors (204) associated with the database (202),
wherein the database (202) is configured to:
- store (S120) plural observation data sets associated with respective observations of chemical, biopharmaceutical, or biotechnological past processes, each of the observation data sets comprising:
-- stored spectral trajectory data,
-- stored process descriptors, and
-- at least one corresponding actual analytical measurement of a process parameter recorded during performance of a respective past process;
wherein the one or more processors (204) are configured to:
- obtain (S140), from a user, user-based input data comprising at least one of the following:
-- input spectral trajectory data, and
-- input process descriptors;
- determine (S160) a subset of the stored spectral trajectory data in accordance with at least one selection criterium by querying the database (202) using the user-based input data; and
- use (S180) the subset of the stored spectral trajectory data to generate a spectral model providing process parameter values for a whole duration of an execution of the process.

10. The computer system of claim 9, wherein the stored spectral trajectory data comprises spectral data or spectra with associated time information, and
wherein the input spectral trajectory data comprises spectral data or spectra with associated time information.

11. The computer system of claim 9 or 10, wherein the one or more processors (204) are configured to control and/or monitor (S200) the process using the spectral model.

12. The computer system of an one of claims 9 to 11, wherein the one or more processors (204) are further configured to:
define the at least one selection criterium based on a similarity between the stored observation data sets and the user-based input data.

13. The computer system of claim 12, wherein the one or more processors (204) are configured to define the selection criterium including a at least one of the following steps (i) to (iii):
(i) matching of the user-based input data with the stored observation data sets;
(ii) spectral pre-processing (applied to the spectral data of the database (202) and to the provided user-based spectral data), by application of at least one of the following methods:
- AsLS correction (Asymmetric Least Square Correction, a non-linear baseline is calculated for each spectrum using symmetric least squares algorithm and then baseline is subtracted from the spectrum),
- moving window (removes noise by applying moving average, or median, window to the spectrum),
- derivatives,
- SNV filter (Standard Normal Variate, each observation (spectra) is "normalized" by subtracting the mean and dividing with the standard deviation),
- MSC (Multiplicative Signal Correction, each spectrum is normalized by regressing it against average spectrum over a selected set of spectra),
- averaging of the (processed) signal over selected signal range,
- peak area, peak height, or water band normalization,
- Multivariate Curve Resolution (MCR);
(iii) comparison of full or parts/phases of process trajectories (by application of at least one of the following methods:
- Comparison of spectral similarities for specific time points or time intervals in the process,
- Dynamic Time Warping,
- Model Arrays,
- TOST test for time series analysis,
- (Spectral) similarity index calculation for the whole process,
- Mechanistic modelling to compare metabolic behavior of processes,
- Batch Modelling - comparison of batch time trajectories using PLS models;
wherein the at least one selection criterium is defined by at least one of the following algorithms:
- maximizing correlation coefficient and minimizing averaged distance for each time point in the trajectory of the Batch Evolution Model,
- minimizing total RMSEP between database batch and user trajectory in the Batch Evolution Model,
- minimizing RMSEP for each time point between database batch and user trajectory in the Batch Evolution Model to identify similarity between individual time points related to process stage, and
- minimizing distance to the center of the Batch Level Model.

14. The computer system of any one of claims 9 to 13, wherein the selection criterium is a 0-1 criterium or a linear criterium.

15. The computer system of any one of claims 9 to 14, wherein the analytical measurements of process parameter comprise a product quality attribute, or concentrations of substrates like glucose, lactate and/or titer.
